# EUROPEAN PATENT SPECIFICATION

(11) **EP 4 609 318 B1**
(45) Date of publication and mention of the grant of the patent: **23.09.2026**
(21) Application number: 24758417.0
(22) Date of filing: 31.07.2024
(51) Int. Cl.: G06N 3/045, G06N 3/048, G06N 3/0985, G06V 10/50, G06V 10/82, G16H 50/20, G16H 50/70, G06N 3/008, G06N 3/044, G06N 3/0464, G06N 3/063, G06N 3/084, G06N 3/088, G06N 3/0895, G06N 3/09

(54) **MIXTURE OF EXPERTS NEURAL NETWORKS WITH SOFT ROUTING**
MISCHUNG AUS NEURONALEN EXPERTS NETZWERKEN MIT WEICHEM ROUTING
RÉSEAUX NEURONAUX DE MÉLANGE D'EXPERTS AVEC ROUTAGE LOGICIEL

(30) Priority: 31.07.2023 US 202363516789 P
(43) Date of publication of application: 03.09.2025
(60) Divisional application: 26198217.7
(73) Proprietor: Google LLC, Mountain View, CA 94043 (US)
(72) Inventor: MUSTAFA, Basil, Mountain View, California 94043 (US); RIQUELME RUIZ, Carlos, Mountain View, California 94043 (US); PUIGCERVER I PEREZ, Joan, Mountain View, California 94043 (US); HOULSBY, Neil Matthew Tinmouth, Mountain View, California 94043 (US)
(74) Representative: Hau, Darren Jun Wai
(86) International application number: PCT/US2024/040407
(87) International publication number: WO 2025/029932

(56) References cited:
- MOHAMMED MUQEETH ET AL: "Soft Merging of Experts with Adaptive Routing", ARXIV.ORG, CORNELL UNIVERSITY LIBRARY, 201 OLIN LIBRARY CORNELL UNIVERSITY ITHACA, NY 14853, 6 June 2023 (2023-06-06), XP091531981
- BRANDON YANG ET AL: "CondConv: Conditionally Parameterized Convolutions for Efficient Inference", ARXIV.ORG, CORNELL UNIVERSITY LIBRARY, 201 OLIN LIBRARY CORNELL UNIVERSITY ITHACA, NY 14853, 4 September 2020 (2020-09-04), XP081755201
- JONAS PFEIFFER ET AL: "Modular Deep Learning", ARXIV.ORG, CORNELL UNIVERSITY LIBRARY, 201 OLIN LIBRARY CORNELL UNIVERSITY ITHACA, NY 14853, 22 February 2023 (2023-02-22), XP091444284
- JOAN PUIGCERVER ET AL: "From Sparse to Soft Mixtures of Experts", ARXIV.ORG, CORNELL UNIVERSITY LIBRARY, 201 OLIN LIBRARY CORNELL UNIVERSITY ITHACA, NY 14853, 27 May 2024 (2024-05-27), XP091766942

## Description

### CROSS REFERENCE TO RELATED APPLICATIONS

This application is a non-provisional of and claims priority to U.S. Provisional Patent Application No. 63/516,789, filed on July 31, 2023.

### BACKGROUND

This specification relates to performing a machine learning task on a network input using neural networks.

Neural networks are machine learning models that employ one or more layers of nonlinear units to predict an output for a received input. Some neural networks include one or more hidden layers in addition to an output layer. The output of each hidden layer is used as input to the next layer in the network, i.e., the next hidden layer or the output layer. Each layer of the network generates an output from a received input in accordance with current values of a respective set of parameters.

Muqeeth, Mohammed, Haokun Liu, and Colin Raffel. "Soft merging of experts with adaptive routing." arXiv preprint arXiv:2306.03745 (2023) discloses that sparsely activated neural networks with conditional computation learn to route their inputs through different "expert" subnetworks, providing a form of modularity that densely activated models lack. They further consider that despite their possible benefits, models with learned routing often underperform their parameter-matched densely activated counterparts as well as models that use non-learned heuristic routing strategies. They hypothesize that these shortcomings stem from the gradient estimation techniques used to train sparsely activated models that use non-differentiable discrete routing decisions. They introduce Soft Merging of Experts with Adaptive Routing (SMEAR), which they disclose avoids discrete routing by using a single "merged" expert constructed via a weighted average of all of the experts' parameters.

Yang, Brandon, et al. "Condconv: Conditionally parameterized convolutions for efficient inference." Advances in neural information processing systems 32 (2019) discloses that one fundamental assumption is that convolutional kernels should be shared for all examples in a dataset. They propose conditionally parameterized convolutions (CondConv), which learn specialized convolutional kernels for each example.

Pfeiffer, Jonas, et al. "Modular deep learning." arXiv preprint arXiv:2302.11529 (2023) provides a survey of modular architectures.

### SUMMARY

This specification describes a system implemented as computer programs on one or more computers in one or more locations that is configured to process a network input using a neural network and to generate a network output characterizing the network input. The scope of the invention is defined by the independent claims. Preferrable embodiments are outlined in the dependent claims.

The subject matter described in this specification can be implemented in particular embodiments so as to realize one or more of the following advantages.

Sparse mixture of expert architectures (MoEs) have been used in neural network models to scale model capacity without large increases in training and inference costs. However, existing systems that use sparse MoEs suffer from a number of issues as described below.

In particular, some existing systems that implement neural network blocks with multiple expert subnetworks use "token-choice" routing, i.e., where the neural network block independently selects, for each element of the block input, a set of one or more expert subnetworks to process the element. Systems that use "token-choice" routing suffer from both "token dropping" (i.e., some tokens are not assigned to any expert) and "load imbalance" (i.e., some expert subnetworks process most or all of the elements of the block input while other expert subnetworks process very few or none of the elements of the block input). Systems that use "expert-choice" routing suffer from the token-dropping issue. As a consequence, performance of these systems can be severely impacted. Load imbalance can result in sub-optimal training because a portion of the network parameters (corresponding to the under-utilized expert subnetworks) do not receive meaningful updates during training and thus do not learn to extract useful information. Furthermore, existing systems that use "token-choice" routing dedicate the same amount of computational resources to each element of the network input, disregarding the relative importance of different elements, which can further reduce the computational efficiency of the systems. The techniques described in this specification overcome the above drawbacks of existing systems because, rather than employing a sparse and discrete router that tries to find a good *hard* assignment between input token and experts, the described techniques perform a *soft* assignment (i.e., soft routing) by mixing input tokens before routing them to each expert subnetwork. In particular, using the described techniques, a system can implement neural network blocks with multiple expert subnetworks using soft routing, i.e., where the expert neural network block computes several weighted averages of all input tokens of the block input with weights depending on both tokens and expert subnetworks, and then processes each weighted average by its corresponding expert subnetwork. Soft routing can ensure that the network block is perfectly load balanced, e.g., by selecting the same number *p* of elements to be processed by each expert subnetwork. The computational and time efficiency of training the neural network can thus be significantly improved. As a result the neural network blocks that employ soft routing can scale to thousands of expert subnetworks and can still be balanced by construction.

In addition, soft routing can eliminate batch-effects at inference, where one input token can affect routing, and hence prediction, for other input tokens. More specifically, when standard (hard) routing is used, tokens are routed in relatively big groups of tokens that can potentially span multiple inputs. For instance, in the case of images, a group of tokens may include tokens (e.g., some or all tokens) from multiple input images (e.g., from four or eight different images) in the batch. In the standard implementation of token-choice and expert-choice (hard) routing algorithms, the decision of which expert (or experts) to use for a given token is taken for the whole group, not independently for each token, which means that tokens from one image can affect which experts are used in tokens from another image. Soft routing works better with small groups when the size of the groups is smaller than or equal to the number of tokens per input (e.g., per image), thereby eliminating the cross-example effect or "contamination". This is very useful especially at inference, e.g. when a model is served in the cloud and queries from independent users are batched together. With the standard (hard) routing algorithms, an user could get non-deterministic behavior (e.g., different outputs) if the user's query is sent twice and batched with other inputs. This problem can be avoided with soft MoEs because soft routing can work with small groups of tokens as described above.

Further, soft routing can allow a network block to more flexibly allocate computational resources to respective elements, e.g., by routing relatively important elements to more expert subnetworks than relatively unimportant elements

In particular, in some implementations in which a system executes different expert subnetworks on respective different devices, the techniques described in this specification allow the system to load balance network inputs to the neural network more efficiently across devices relative to existing techniques (e.g., relative to systems that implement token-choice routing). The inferior load balancing that the existing systems suffer because of the token-choice routing can harm inference performance (e.g., by reducing computational and/or memory efficiency or by increasing the amount of time required to generate a network output) because different devices executing different expert subnetworks can have significantly different loads, and thus some devices can be underutilized while others can be overworked. Using soft routing as described in this specification, perfect load balancing can be "baked in" at inference time, the system can enjoy significantly improved performance (e.g., increased computational and/or memory efficiency or decreased time required to generate a network output) across the multiple devices because the multiple devices each have a similar or equivalent amount of operations to execute. Therefore, relative to other existing approaches, the described approach results in a system that can process inputs at a higher throughput relative to conventional approaches by being optimized for a distributed hardware implementation.

Further, systems that use soft routing as described herein are immune to the "token dropping" issue, which systems that use "expert choice" routing and "token-choice" routing suffer from. This is because soft routing computes weighted averages of all input tokens before routing them to the corresponding expert subnetworks. As all tokens are considered for the *soft* assignment, there will not be any tokens that are not assigned to any expert subnetwork.

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1 illustrates an example neural network system that includes one or more network blocks.
FIG. 2A illustrates a comparison between an existing sparse MoE router and the soft MoE router described in this specification.
FIG. 2B shows example operations of an expert network block that uses soft routing.
FIG. 3 is a flow diagram of an example process for processing a block input to generate a block output.
FIG. 4A illustrates a comparison of performance of a soft MoE model and other models including a ViT model, a token-choice routing-based model, and an expert-choice routing-based model using the upstream validation precision-at-1 metric.
FIG. 4B illustrates a comparison of performance of a soft MoE model and other models including a ViT model, a token-choice routing-based model, and an expert-choice routing-based model using the ImageNet 10-shot accuracy metric.
FIG. 5 is a table that shows different Vision Transformer (ViT) models and soft MoE models.
FIG. 6 shows performance of ViT models and soft MoE models with long training durations.
FIG. 7 shows performance of ViT and soft MoE models when they are optimized for inference speed.

Like reference numbers and designations in the various drawings indicate like elements.

### DETAILED DESCRIPTION

This specification describes a system implemented as computer programs on one or more computers in one or more locations that performs a machine learning task on a network input to generate a network output for the machine learning task.

The machine learning task can be any machine learning task that operates on a network input that is an input sequence, i.e., a collection of multiple elements, to generate a network output for the network input.

Some examples of machine learning tasks that the system can be configured to perform are described as follows.

As an example, the task can be a natural language processing or understanding task, e.g., an entailment task, a paraphrase task, a textual similarity task, a sentiment task, a sentence completion task, a grammaticality task, and so on, that operates on a sequence of text in some natural language to generate classification output that classifies the text into one or more categories from a set of categories.

As another particular example, the task can be a text generation task, where the input is a sequence of text, and the output is another sequence of text, e.g., a completion of the input sequence of text, a response to a question posed in the input sequence, or a sequence of text that is about a topic specified by the first sequence of text. For instance, the neural network can be an autoregressive neural network, e.g., a self-attention based autoregressive neural network. As another example, the input to the text generation task can be an input other than text, e.g., an image, and the output sequence can be text that describes the input.

As another particular example, the task can be a code generation task, i.e., generating computer code in response to a context input. The context input may include computer code, or natural language text, or both, and the output is another computer code.

As an example, the task may be an audio processing task. For example, if the input to the neural network is a sequence representing a spoken utterance, the output can be a classification output that classifies the spoken utterance into one or more categories from a set of categories. For example, if the input to the neural network is a sequence representing a spoken utterance, the output generated by the neural network can indicate whether a particular word or phrase ("hotword") was spoken in the utterance. As another example, if the input to the neural network is a sequence representing a spoken utterance, the output generated by the neural network can identify the natural language in which the utterance was spoken. It will be understood that in the case of an audio processing task, the input to the neural network may comprise audio data (e.g. an audio signal), for example in the form of a sequence of audio data frames, and that the audio data may be processed to perform the audio processing task.

As an example, the network input can represent a sequence of audio data, and the machine learning task may be a speech recognition task, where the neural network is configured to process a representation of an audio waveform to generate an output that characterizes a sequence of phonemes, characters, or words corresponding to the audio waveform.

As another example, the task can be a health prediction task, where the input is a sequence derived from electronic health record data for a patient and the output is a prediction that is relevant to the future health of the patient, e.g., a predicted treatment that should be prescribed to the patient, the likelihood that an adverse health event will occur to the patient, or a predicted diagnosis for the patient.

As another example, the task can be an agent control task, where the input is a sequence of observations or other data characterizing states of an environment and the output defines an action to be performed by the agent in response to the most recent data in the sequence. The agent can be, e.g., a real-world or simulated robot, a control system for an industrial facility, or a control system that controls a different kind of agent.

As another example, the task can be a genomics task, where the input is a sequence representing a fragment of a DNA sequence or other molecule sequence and the output is either an embedding of the fragment for use in a downstream task, e.g., by making use of an unsupervised learning technique on a data set of DNA sequence fragments, or an output for the downstream task. Examples of downstream tasks include promoter site prediction, methylation analysis, predicting functional effects of non-coding variants, and so on.

As another example, the task can be a computer vision task, where the input is an image or a point cloud and the output is a computer vision output for the image or point cloud. It will be understood that the image may comprise pixel data, which may be processed to perform the computer vision task. The neural network can be configured to process images of any appropriate type, e.g., RGB images, LIDAR images (e.g., point clouds), and so on.

For example, the computer vision task can be a classification task that requires generating a classification output. A classification output generally includes a respective score corresponding to each of multiple categories. The score for a category indicates a likelihood that the image belongs to the category. In some cases, the categories may be classes of objects (e.g., dog, cat, person, and the like), and the image may belong to a category if it depicts an object included in the object class corresponding to the category. In some cases, the categories may represent global image properties (e.g., whether the image depicts a scene in the day or at night, or whether the image depicts a scene in the summer or the winter), and the image may belong to the category if it has the global property corresponding to the category.

As another example, the computer vision task can be an object detection task. In an object detection task, the output generated by the neural network identifies locations, e.g., bounding boxes or other regions, in the input image at which particular types of objects are depicted.

As another example, the computer vision task can be an instance segmentation task. In an instance segmentation task, the output generated by the neural network identifies, for each pixel in the image that belongs to a particular object type, the object instance that the pixel corresponds to.

As another example, the computer vision task can be a semantic segmentation task. In a semantic segmentation task, the output generated by the neural network identifies, for each pixel in the image, which of multiple categories the pixel belongs to.

As another example, the computer vision task can be a depth prediction task. In a depth prediction task, the output generated by the neural network identifies, for each pixel in the image, a predicted depth of the scene at the pixel.

As another example, the computer vision task can be a surface normal prediction task. In a surface normal prediction task, the output generated by the neural network identifies, for each pixel in the image, a predicted surface normal of the scene at the pixel.

When the input is an image or point cloud, the neural network can include an embedding subnetwork that generates a respective embedding for each multiple patches of the image or point cloud, and the input to the first block of the neural network can be a sequence that includes the respective embeddings (and, optionally, one or more additional embeddings, e.g., at a predetermined position that will later be used to generate the output). Each patch includes the intensity values of the pixels in a different region of the input image.

As another particular example, the neural network can be configured to generate a regression output that estimates one or more continuous variables (i.e., that can assume infinitely many possible numerical values) that characterize the network input. In a particular example, if the network input represents an image, the regression output may estimate the coordinates of bounding boxes that enclose respective objects depicted in the image. The coordinates of a bounding box may be defined by (x, y) coordinates of the vertices of the bounding box.

As another example, the network input can represent a sequence of video frames, and the machine learning task may be a video analysis task, where the neural network is configured to process a sequence of video frames to generate an output that characterizes the video frames, e.g., by characterizing whether the video frames depict a person performing a particular action.

In some implementations, the task is a multi-modal task that requires processing both text and image inputs, so that the neural network includes both a computer vision neural network and a text processing neural network. That is, the target output to be generated by the computer vision neural network for a given image depends on one or more outputs generated by the text processing neural network for one or more corresponding text inputs (and vice versa). Examples of such tasks include open-vocabulary image classification, open-vocabulary object detection, image captioning, text-based image search, image-based retrieval, and so on.

In some cases, the machine learning task is a combination of multiple individual machine learning tasks, i.e., the system is configured to perform multiple different individual machine learning tasks, e.g., two or more of the machine learning tasks mentioned above. For example, the system can be configured to perform multiple individual natural language understanding tasks, with the network input including an identifier for the individual natural language understanding task to be performed on the network input.

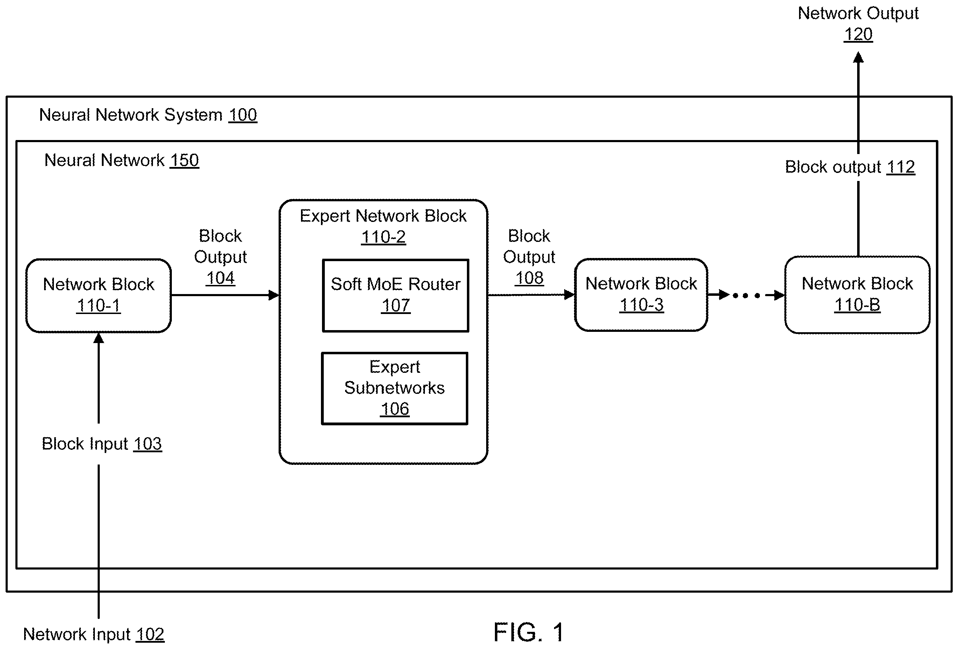

FIG. 1 illustrates an example neural network system 100. The system 100 is an example of a system implemented as computer programs on one or more computers in one or more locations, in which the systems, components, and techniques described below can be implemented.

The system 100 is a system that processes a network input 102 using a neural network 150 to generate a network output 120 characterizing the network input 102 for a machine learning task, e.g., one of the tasks described above.

The neural network 150 includes a sequence of one or more network blocks (e.g., network blocks 110-1, 110-2, , ..., 110-B) that are each configured to process a block input that includes the network input or an intermediate representation of the network input and to generate a block output.

A "network block," as used in this specification, is a collection of one or more neural network layers that receive an input ("a block input") and process the input to generate an output (a "block output").

For example, the first network block in the sequence of network blocks (e.g., network block 110-1) can process the block input 103. In some implementations, the block input 103 is the same as the network input 102. In some other implementations, the block input 103 is an intermediate representation of the network input 102. For example, the block input 103 can be embeddings of the network input 102 generated by an embedding subnetwork. Each subsequent network block can then process the block output of the previous network block in the sequence. For example, the network block 110-2 takes the block output 104 of the network block 110-1 as its block input and processes the block output 104 to generate the block output 108.

In some implementations, the network output 120 for the neural network 150 is the block output 112 of the final network block 110-B in the sequence.

In some other implementations, the block output 112 of the final network block 110-B in the sequence is further processed using one or more output neural network layers to generate the network output 120 for the neural network 150.

The sequence of network blocks can include one or more "expert" network blocks, e.g., network block 110-2 is an expert network block. Although FIG. 1 shows one expert network block 110-2, the sequence of network blocks may include multiple expert network blocks, e.g., two expert network blocks, five expert network blocks, or tens, hundreds or thousands of expert network blocks. Each expert network block includes multiple different expert subnetworks 106 that are each configured to process respective input tokens determined from the block input to the expert network block. As discussed above, the block input to each expert network block is the network input or represents an intermediate representation of the network input and the block input includes a plurality of input tokens. All of the network blocks in the sequence can be expert network blocks or a subset of the network blocks in the sequence can be expert network blocks.

For each of the expert subnetworks 106 of the expert network block 110-2, a soft mixtures of experts (MoE) router 107 within the expert network block 110-2 is configured to perform a *soft* assignment (i.e., soft routing) by assigning different weighted combinations of all input tokens to each expert subnetwork. By using soft routing, the expert subnetworks only process a subset of the (combined) tokens, enabling larger model capacity and performance at lower inference cost. More specifically, each expert processes a total number of slots that is less than the total number of input tokens. However, each slot is a weighted combination of all of the input tokens, ensuring that each expert obtains information from all of the input tokens. Thus, this enables larger model capacity and performance, i.e., due to different experts being able to process different weighted combinations of the inputs, at lower inference cost, i.e., because each expert only needs to process a small number of input slots even when there is a large number of input tokens.

In particular, the soft MoE router 107 is configured to generate a plurality of input slots from the plurality of input tokens of the block input. Each input slot is associated with a respective set of dispatch weights. For each input slot, the soft MoE router 107 is configured to combine the plurality of input tokens in accordance with the respective set of dispatch weights for the input slot to generate the input slot. Thus, each input slot is a different weighted combination of the plurality of input tokens.

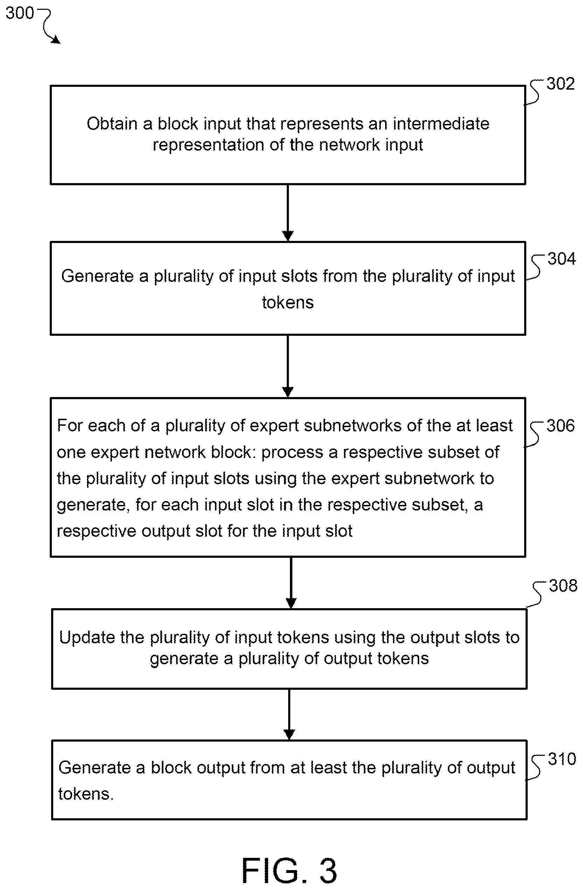

Each of the plurality of expert subnetworks in the expert network block 110-2 is then configured to process a respective subset of the plurality of input slots to generate, for each input slot in the respective subset, a respective output slot for the input slot. The operations performed by the expert subnetworks and example architectures for the expert subnetworks are described in further detail below with reference to FIG. 3.

The expert network block 110-2 then updates the plurality of input tokens using the output slots to generate a plurality of output tokens.

The expert network block 110-2 can then combine the plurality of output tokens to generate the block output 108 for the expert network block 110-2. For example, the expert network block 110-2 can concatenate the plurality of output tokens, e.g., in the same configuration (e.g., in the same order) as the corresponding plurality of input token in the block input of the expert network block 110-2.

Optionally, as part of combining the output tokens, the system can apply one or more additional operations to the concatenation of the output tokens, e.g., the concatenation may be processed by one or more of feed-forward layers, skip connections, or normalization operations, e.g., layer normalization, to provide the block output.

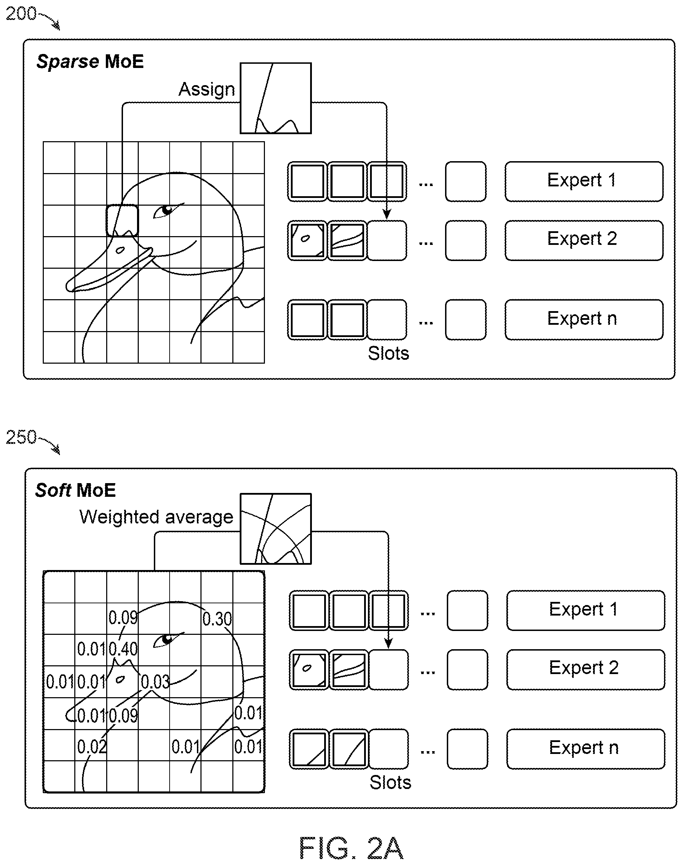

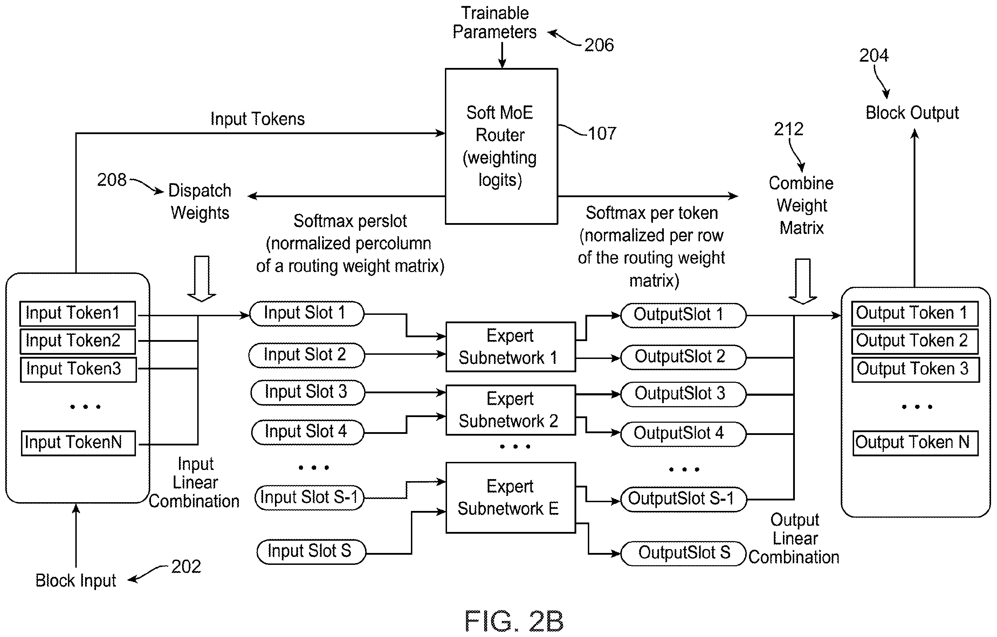

Soft routing is described in more detail below with reference to FIG. 2A and FIG. 2B.

The input tokens can be any appropriate subset of the elements of the block input. For example, if the neural network is configured to process an input sequence (e.g., an input sequence representing an image, text data, or audio data), then each block input can be an intermediate sequence that is an intermediate representation of the input sequence, and the input tokens can be subsequences of the intermediate sequence.

In some implementations, each input token is the same size, i.e., includes the same number of elements. For example, each input token can be a different one of the elements in the block input. In some other implementations, different input tokens can be different sizes, i.e., include different numbers of elements.

In some implementations, each element of the block input is in exactly one input token. In some other implementations, some or all of the elements of the block input can be in multiple different input tokens.

The operations performed by the expert network blocks 110-2 are described in more detail below with reference to FIGS. 2 and 3.

In some implementations, the sequence of network blocks (e.g., network blocks 110-1, 110-2, ... 110-B) includes one or more expert network blocks interspersed among other types of network blocks, e.g., self-attention network blocks that apply self-attention, that do not include routers and expert neural networks, i.e., that do not perform conditional computation and use all of the parameters of network block for all inputs to the network block. As a particular example, the sequence of network blocks can alternate between expert network blocks and self-attention network blocks. As another particular example, the sequence of network blocks can include self-attention network blocks, feed-forward network blocks that include a single neural network that has the same architecture as the expert neural networks and that processes all of the input tokens in the block input to the feed-forward block, and expert network blocks. For example, every other self-attention network block in the sequence can be immediately followed by an expert network block 110-2, with the remainder of the self-attention network blocks being followed by a feed-forward network block.

Each self-attention network block is configured to process a block input using one or more self-attention neural network layers.

A self-attention neural network layer receives as input a sequence of input elements and applies an attention mechanism over the sequence of input elements to generate a sequence of layer outputs elements. In particular, for each input element, the self-attention neural network layer applies the attention mechanism over the sequence of input elements using one or more queries derived from the input element to generate a respective output element. Some self-attention neural network layers are multi-head self-attention neural network layers. A multi-head self-attention neural network layer applies h different attention mechanisms in parallel to generate respective sequences of output elements, and then combines the multiple sequences of output elements to generate a final sequence of output elements.

Self-attention is described in more detail below.

The expert network block 110-2 is implemented such that the expert subnetworks of the expert network block 110-2 are executed in parallel for a given block input, thus improving the efficiency of the system. For example, the expert network block 110-2 can be implemented on a parallel processing device, e.g., a GPU or a TPU, that can execute the expert subnetworks on respective different threads. In addition or alternatively, at least some expert subnetworks 106 of the expert network block 110-2 can be implemented on respective different devices, e.g., different devices that are communicatively connected and that provide the output tokens generated by the respective expert subnetwork to a single device for combining to generate the respective combined output tokens.

Thus, the network architecture of a neural network that includes one or more expert network blocks each having multiple expert subnetworks is optimized for efficient execution of the neural network. Such a network architecture allows the operations of the neural network to be parallelized for quick and low-cost execution, e.g., by parallelizing the operations of respective expert subnetworks across multiple devices. The neural network can thus be implemented on dedicated parallel processing hardware, e.g., a network of multiple parallel processing devices that each execute respective expert subnetworks of the neural network.

As will be described below, by implementing soft routing, the system 100 optimizes the parallelization of the processing that is performed for each network input.

FIG. 2A illustrates a comparison between a sparse MoE architecture 200 and a soft MoE architecture 250 as described in this specification. In the example of FIG. 2A, both architectures (e.g., expert network blocks) receive an image of a bird as a block input. The block input has a plurality of input tokens. A sparse MoE router in the architecture 200 assigns *individual* input tokens to each of the available input slots. Rather than trying to find a good *hard* assignment between tokens and experts like the sparse MoE router, the soft MoE router of the architecture 250 instead performs a *soft* assignment by mixing input tokens before routing. In particular, the soft MoE router computes, for each input slot, a (different) *weighted average* of all of the input tokens. Each of these input slots is then processed by a corresponding expert in the plurality of experts (e.g., experts 1, 2,...n). FIG. 2B shows example operations of an expert network block that uses soft routing. The expert network block 110-2 includes a soft MoE router 107 and a plurality of expert subnetworks (e.g., expert subnetworks 1, 2, ..., S-1, S).

In particular, in the example of FIG. 2B, the expert network block 110-2 is processing a plurality of input tokens (e.g., input token 1, 2, 3, ...N) of a block input 202 to generate a block output 204.

The soft MoE router 107 of the expert network block 110-2 generates a plurality of input slots (e.g., input slot 1, input slot 2, ..., input slot S) from the plurality of input tokens. More specifically, each input slot is associated with a respective set of dispatch weights 208.

To compute the dispatch weights 208 for a given slot, the soft MoE router 107 applies a set of trainable , i.e., learned, parameters for the slot to the input tokens.

For example, the router 107 can compute a routing weight matrix by taking a product of the plurality of input tokens and the trainable parameters 206 for all input slots. The trainable parameters 206 can be learned through a joint training with the soft MoE router and the expert subnetworks in the expert block 110-2, as described in further detail below. The soft MoE router 107 then computes the dispatch weights 208 for the slots by applying a softmax over the columns of the routing weight matrix. The routing weight matrix, denoted as *R,* is a product of the plurality of input tokens *X* ∈ *R*^{*m*×*d*} (where *m* is the number of input tokens and *d* is the dimension of each input token) and the trainable parameters 206 for the input slots, denoted as, *ϕ* ∈ *R*^{*d×*(*n.p*)}*.* Each input slot has a corresponding vector in the *d-*dimensional vector of trainable parameters *ϕ*.

In particular, the soft MoE router 107 generates a dispatch weight matrix *D* for the input slots by applying a softmax over columns of a routing weight matrix *L* as follows: ${D}_{i , j} = \frac{exp \left({L}_{i , j}\right)}{{\sum }_{i ′ = 1}^{m} exp \left({L}_{i ′ , j}\right)} ,$ wherein *i* and *i'* are row indices of the dispatch weights matrix D and the routing weight matrix *L, j* is the column index of the dispatch weight matrix D and the routing weight matrix *L.* The routing weight matrix *L* is a product of the plurality of input tokens *X* ∈ *R*^{*m*×*d*} (where *m* is the number of input tokens and *d* is the dimension of each input token) and the trainable parameters 206 for the slots, *ϕ* ∈ *R*^{*d×*(*n.p*)}*.*

The soft MoE router 107 computes each input slot by combining the plurality of input tokens in accordance with the respective set of dispatch weights of the input slot. For example, to generate each input slot, the soft MoE router 107 takes a weighted average of the plurality of input tokens using the respective set of dispatch weights of the input slot in the dispatch weight matrix D.

In particular, the soft MoE router 107 computes the input slots *X̃* ∈ *R*^{(*n.p*)×*d*} which are the result of convex combinations of all of the *m* input tokens, *X:* ${X}^{˜} = {D}^{T} X ,$ where *D^{T}* is a transpose of the dispatch weight matrix *D* that includes a respective set of dispatch weights for each input token.

For each of the plurality of expert subnetworks, the expert network block 110-2 processes a respective subset of the plurality of input slots using the expert subnetwork to generate, for each input slot in the respective subset, a respective output slot for the input slot. The corresponding expert function of each expert subnetwork is applied on each of the slots assigned to that expert subnetwork (i.e. on rows of *X̃*) to obtain the output slots *Ỹ.*

In particular, the expert network block computes a respective output slot *Ỹᵢ* as follows: ${{Y}^{˜}}_{i} = {f}_{\left⌊i/p\right⌋} \left({{X}^{˜}}_{i}\right) ,$ where *f* denotes the expert subnetwork (or the corresponding expert function), *X̃ᵢ* represents each input slot in the respective subset of *p* input slots processed by the expert subnetwork, and *Ỹᵢ* is the respective output slot generated by the expert subnetwork for the input slot *X̃ᵢ.*

Each expert subnetwork is configured to process *p* slots in the plurality of input slots. The respective input slots processed by each expert subnetwork is predetermined (i.e., the mapping between slots and experts is fixed). The input slots processed by one expert subnetwork does not overlap with the input slots processed by other expert subnetworks. For example, in FIG. 2B, each subnetwork processes 2 slots. The expert subnetwork 1 processes input slot 1 and input slot 2 to generate output slot 1 and output slot 2, respectively. Similarly, the expert subnetwork 2 processes input slot 3 and input slot 4 to generate output slot 3 and output slot 4, respectively, and so on.

After generating output slots, the expert network block updates the plurality of input tokens using the output slots to generate a plurality of output tokens (e.g., output tokens 1, 2,..., N).

The soft MoE router 107 first computes a combine weight matrix 212 by applying a softmax over rows of the routing weight matrix. The combine weight matrix 212 includes a respective set of combine weights for each input token.

For each of the plurality of input tokens, the expert network block combines the output slots generated by all expert subnetworks in accordance with the respective set of combine weights associated with the input token to generate a respective output token. The expert network block then generates a block output 204 from the plurality of output tokens 1, 2, ..., N.

In some implementations, the expert network block can concatenate the output tokens, e.g., in the same configuration (e.g., in the same order) as the corresponding input tokens in the block input.

Optionally, as part of combining the output tokens, the system can apply one or more additional operations to the concatenation of the output tokens, e.g., the concatenation may be processed by one or more of feed-forward layers, skip connections, or normalization operations, e.g., layer normalization, to provide the block output.

In some implementations, the expert network block generates the block output from at least the plurality of output tokens by applying a residual connection to the plurality of output tokens to generate the block output.

In some other implementations, the expert network block generates a block output from at least the plurality of output tokens by using one or more neural network layers, e.g., one or more self-attention layers, one or more convolutional neural network layers, and/or one or more recurrent neural network layers.

Existing systems that use traditional MoE routing methods (e.g., "token choice" routing) can suffer from load imbalance, where some expert subnetworks process most or all of the elements of the block input while other expert subnetworks process very few or none of the elements of the block input. Such load imbalance can result in sub-optimal training because a portion of the network parameters (corresponding to the under-utilized expert subnetworks) do not receive meaningful updates during training and thus do not learn to extract useful information. Furthermore, this system dedicates the same amount of computational resources to each element of the network input, disregarding the relative importance of different elements, which can further reduce the computational efficiency of the systems.

In contrast, as can be seen from FIG. 2B, soft routing can ensure that the expert network block is perfectly load balanced, e.g., by computing weighted combinations of input tokens before routing them to each expert subnetwork. This soft routing can further increase load balancing by selecting the same number *p* of input slots to be processed by each expert subnetwork. The computational and time efficiency of training the neural network can thus be significantly improved. As a result, the neural network blocks that employ soft routing can scale to thousands of expert subnetworks and can still be balanced by construction. In addition, soft routing can reduce batch-effects at inference, where one input token can affect routing, and hence prediction, for other input tokens. Further, soft routing can allow a network block to more flexibly allocate computational resources to respective elements, e.g., by routing relatively important elements to more expert subnetworks than relatively unimportant elements.

In particular, in some implementations in which a system executes different expert subnetworks on respective different devices, the techniques described in this specification allow the system to load balance network inputs to the neural network more efficiently across devices relative to existing techniques (e.g., relative to systems that implement token-choice routing). The inferior load balancing that the existing systems suffer because of the token-choice routing can harm inference performance (e.g., by reducing computational and/or memory efficiency or by increasing the amount of time required to generate a network output) because different devices executing different expert subnetworks can have significantly different loads, and thus some devices can be underutilized while others can be overworked. Using soft routing as described in this specification, perfect load balancing can be "baked in" at inference time, the system can enjoy significantly improved performance (e.g., increased computational and/or memory efficiency or decreased time required to generate a network output) across the multiple devices because the multiple devices each have a similar or equivalent amount of operations to execute. Therefore, relative to other existing approaches, the described approach results in a system that can process inputs at a higher throughput relative to conventional approaches by being optimized for a distributed hardware implementation.

Further, because each of the *p* input slots processed by each expert subnetwork is a combination of all of the input tokens, no information is lost when the expert network block processes a block input. This is because each expert subnetwork obtains information from all input tokens (with each input token being weighted differently). Thus, the soft routing can maintain perfect load balancing while ensuring high output quality.

Processing a block input using soft routing is described in more detail below with reference to FIG. 3.

FIG. 3 is a flow diagram of an example process 300 for processing a block input to generate a block output. For convenience, the process 300 will be described as being performed by a system of one or more computers located in one or more locations. For example, an expert network block included in a neural network system, e.g., the expert network block 110-2 included in the neural network system 100 of FIG. 1, appropriately programmed in accordance with this specification, can perform the process 300.

The expert network block is configured to obtain a block input that represents an intermediate representation of the network input (step 302). The block input includes a plurality of input tokens. Each input token includes a respective different subset of a plurality of elements of the block input. In some implementations, each input token includes exactly one of the elements of the block input, i.e., each input token is a different one of the elements of the block input.

More generally, in some implementations, each input token is the same size, i.e., includes the same number of elements. In some other implementations, different input tokens can be different sizes, i.e., include different numbers of elements.

In some implementations, each element of the block input is in exactly one input token. In some other implementations, some or all of the elements of the block input can be in multiple different input tokens.

The expert network block then performs steps 304-308 for each of a plurality of expert subnetworks of the expert network block.

The expert network block, i.e., the soft MoE router within the expert network block, generates a plurality of input slots from the plurality of input tokens (step 304). Each input slot is associated with a respective set of dispatch weights. The expert network block generates the plurality of input slots by combining the plurality of input tokens in accordance with the respective set of dispatch weights.

In particular, the input tokens for one block input can be denoted as *X* ∈ *R*^{*m*×}*^{d},* where *m* is the number of input tokens and *d* is the dimension of each input token. Each expert network block includes a set of *n* expert subnetworks each corresponding to an expert function (which will be applied to the respective set of input slots processed by the expert subnetwork). The expert subnetworks (or expert functions) can be denoted as {*fᵢ:R^{d}* → *R^{d}*}_{1:}*ₙ.*

Each expert subnetwork is configured to process *p* input slots, and each input slot has a corresponding *d*-dimensional vector of trainable parameters, so that the vectors for all of the input slots form a matrix *ϕ* ∈ *R*^{*d×*(*n.p*)}*.* The number of input slots is (n.p), which is a key hyperparameter of the expert network block because the time complexity depends on the number of slots rather than on the number of experts.

Each expert subnetwork can include one or more feedforward neural network layers, one or more convolutional neural network layers, one or more recurrent neural network layers, and/or one or more self-attention neural network layers. In some implementations, all expert subnetworks may have the same architecture (e.g., the same layers) and may apply the same expert function *f*(with different parameters) to their respective input slots. In some other implementations, each expert subnetwork may apply a different expert function to its corresponding input slots.

The expert network block computes the input slots *X̃* ∈ *R*^{(*n.p*)×*d*} which are the result of convex combinations of all of the *m* input tokens, *X:* ${X}^{˜} = {D}^{T} X ,$ where *D^{T}* is a transpose of a dispatch weight matrix D that includes a respective set of dispatch weights for each input token.

The expert network block, i.e., the soft MoE router within the expert network block, generates the dispatch weight matrix *D* by applying a softmax over columns of a routing weight matrix *L* as follows: ${D}_{i , j} = \frac{exp \left({L}_{i , j}\right)}{{\sum }_{i ′ = 1}^{m} exp \left({L}_{i ′ , j}\right)} ,$ wherein *i* and *i'* are row indices of the dispatch weights matrix D and the routing weight matrix *L,* j is the column index of the dispatch weight matrix D and the routing weight matrix *L.*

The routing weight matrix *L* is a product of the plurality of input tokens *X* and a set of trainable parameters *ϕ* ∈ *R*^{*d*×(*n.p*)}: $L = Xϕ ,$

For each of the plurality of expert subnetworks, the expert network block processes a respective subset of the plurality of input slots using the expert subnetwork to generate, for each input slot in the respective subset, a respective output slot for the input slot (step 306).

In particular, the expert network block computes a respective output slot *Ỹᵢ* as follows: ${{Y}^{˜}}_{i} = {f}_{\left⌊i/p\right⌋} \left({{X}^{˜}}_{i}\right) ,$ where *f* is the expert subnetwork (or the corresponding expert function), *X̃ᵢ* represents each input slot in the respective subset of *p* input slots processed by the expert subnetwork, and *Ỹᵢ* is the respective output slot generated by the expert subnetwork for the input slot *X̃ᵢ*.

The expert network block updates the plurality of input tokens using the output slots to generate a plurality of output tokens (step 308). In particular, for each of the plurality of input tokens, the expert network block generates a respective output token. Each input token is associated with a respective set of combine weights. The expert network block combines the output slots generated by the plurality of expert subnetworks in accordance with the respective set of combine weights associated with the input token to generate the respective output token for the input token, as shown in the following equation: $Y = C {Y}^{˜} ,$ where *Ỹ* represents the output slots, *C* is a combine weight matrix that includes a respective set of combine weights for each input token, and Y represents the plurality of output tokens.

The expert network block (i.e., the soft MoE router within the expert network block) generates the combine weight matrix C by applying a softmax over rows of the routing weight matrix L as follows: ${C}_{i , j} = \frac{exp \left({L}_{i , j}\right)}{{\sum }_{j ′ = 1}^{n . p} exp \left({L}_{i , j ′}\right)} ,$ wherein *i* is the row index of the combine weight matrix *C* and the routing weight matrix *L, j* and *j*' are column indices of combine weight matrix *C* and the routing weight matrix *L.*

The expert network block generate a block output from at least the plurality of output tokens (step 310).

In some implementations, the expert network block can concatenate the output tokens, e.g., in the same configuration (e.g., in the same order) as the corresponding input tokens in the block input.

Optionally, as part of combining the output tokens, the system can apply one or more additional operations to the concatenation of the output tokens, e.g., the concatenation may be processed by one or more of feed-forward layers, skip connections, or normalization operations, e.g., layer normalization, to provide the block output.

In some implementations, the expert network block generates the block output from at least the plurality of output tokens by applying a residual connection to the plurality of output tokens to generate the block output.

In some other implementations, the expert network block generates a block output from at least the plurality of output tokens by using one or more neural network layers, e.g., one or more self-attention layers, one or more convolutional neural network layers, and/or one or more recurrent neural network layers.

In some implementations, expert network blocks can be used to replace a subset of the Transformer's MLP blocks. In these implementations, the number of slots can be set to equal to the length of an input sequence to match the FLOPs of the equivalent dense Transformer.

The expert network block is implemented such that the expert subnetworks of the expert network block are executed in parallel for a given block input, thus improving the efficiency of the system. For example, the expert network block can be implemented on a parallel processing device, e.g., a GPU or a TPU, that can execute the expert subnetworks on respective different threads. In addition, or alternatively, at least some expert subnetworks of the expert network block can be implemented on respective different devices, e.g., different devices that are communicatively connected and that provide the sub-outputs generated by the respective expert subnetwork to a single device for combining to generate the respective combined sub-outputs.

Thus, the network architecture of a neural network that includes expert network blocks with multiple expert subnetworks is optimized for efficient execution of the neural network. Such a network architecture allows the operations of the neural network to be parallelized for quick and low-cost execution, e.g., by parallelizing the operations of respective expert subnetworks across multiple devices. The neural network can thus be implemented on dedicated parallel processing hardware, e.g., a network of multiple parallel processing devices that each execute respective expert subnetworks of the neural network.

In some implementations the sequence of network blocks includes one or more network blocks that are not expert network blocks. For example, the sequence of network blocks can include one or more self-attention network blocks that are configured to process a block input using one or more self-attention neural network layers. As a particular example, the sequence of network blocks can alternate between expert network blocks and self-attention network blocks.

A self-attention neural network layer receives as input a sequence of input elements and applies an attention mechanism over the sequence of input elements to generate a sequence of layer outputs elements. In particular, for each input element, the self-attention neural network layer applies the attention mechanism over the sequence of input elements using one or more queries derived from the input element to generate a respective output element. Some self-attention neural network layers are multi-head self-attention neural network layers. A multi-head self-attention neural network layer applies *h* different attention mechanisms in parallel to generate respective sequences of output elements, and then combines the multiple sequences of output elements to generate a final sequence of output elements.

The expert network block is implemented such that the expert subnetworks of the expert network block are executed in parallel for a given block input, thus improving the efficiency of the system. For example, the expert network block can be implemented on a parallel processing device, e.g., a GPU or a TPU, that can execute the expert subnetworks on respective different threads. In addition, or alternatively, at least some expert subnetworks of the expert network block can be implemented on respective different devices, e.g., different devices that are communicatively connected and that provide the sub-outputs generated by the respective expert subnetwork to a single device for combining to generate the respective combined sub-outputs.

Thus, the network architecture of a neural network that includes expert network blocks with multiple expert subnetworks is optimized for efficient execution of the neural network. Such a network architecture allows the operations of the neural network to be parallelized for quick and low-cost execution, e.g., by parallelizing the operations of respective expert subnetworks across multiple devices. The neural network can thus be implemented on dedicated parallel processing hardware, e.g., a network of multiple parallel processing devices that each execute respective expert subnetworks of the neural network.

Prior to using the neural network to perform the machine learning task, a training system trains the neural network to perform the task, i.e., to determine trained values of the parameters of the neural network, i.e., of the blocks in the sequence, and, optionally, an embedding subnetwork used to generate the input to the first block in the sequence, an output subnetwork that generates the network output from the output of the last block in the sequence, or both. For example, the training system can train the neural network from scratch on training data for the task to minimize a loss function for the task, e.g., a cross-entropy loss, a negative log likelihood loss, and so on using conventional machine learning techniques. As another example, the training system can first pre-train the neural network on an unsupervised objective and then fine-tune the neural network on the training data for the task. As yet another example, the training system can train the neural network on both unlabeled data and the training data for the task through semi-supervised learning.

Because the system employs soft routing in which load balancing can be "baked-in," the system does not need to utilize any auxiliary losses that encourage load balancing across experts during training, improving the stability and efficiency of training relative to conventional approaches.

Moreover, by making use of soft routing and training the soft MoE router of each expert block by backpropagating gradients of the overall loss, the system allows each expert subnetwork to be trained, i.e., to become configured through training, to process different types of network inputs, allowing the expert subnetworks to "specialize" and further improving the efficiency and performance of the neural network. The trainable parameters for input slots can be learned through this joint training process.

During training, the training system can incorporate any number of techniques to improve the speed, the effectiveness, or both of the training process. For example, the system can use dropout, label smoothing, or both to reduce overfitting. As another example, the system can perform the training using a distributed architecture that trains multiple instances of the neural network in parallel. Moreover, as described above, the system can first pre-train the neural network on a large unsupervised data set through unsupervised learning, e.g., to minimize a BERT loss or other unsupervised loss, and then fine-tune the neural network on task-specific training data to optimize the loss function for the task.

An "embedding," as used in this specification is a vector of numeric values, e.g., floating point or other type of numeric values, that has a predetermined dimensionality, e.g., has a predetermined number of values.

A self-attention block, as referred to above, is a neural network layer that includes an attention mechanism that operates over the self-attention block input (or an input derived from the layer input) to generate the self-attention block output. A self-attention mechanism may be causally masked so that any given position in an input sequence does not attend over (e.g. use data from) any positions after the given position in the input sequence. There are many different possible attention mechanisms. Some examples of self-attention layers including attention mechanisms, are described in Vaswani et al. "Attention is all you need", 31st Conference on Neural Information Processing Systems (NIPS 2017), Long Beach, CA, USA; Colin Raffel, Noam Shazeer, Adam Roberts, Katherine Lee, Sharan Narang, Michael Matena, Yanqi Zhou, Wei Li, and Peter J Liu. Exploring the limits of transfer learning with a unified text-to-text transformer. arXiv preprint arXiv:1910.10683, 2019; Daniel Adiwardana, Minh-Thang Luong, David R. So, Jamie Hall, Noah Fiedel, Romal Thoppilan, Zi Yang, Apoorv Kulshreshtha, Gaurav Nemade, Yifeng Lu, and Quoc V. Le. Towards a human-like open-domain chatbot. CoRR, abs/2001.09977, 2020; and Tom B Brown, Benjamin Mann, Nick Ryder, Melanie Subbiah, Jared Kaplan, Prafulla Dhariwal, Arvind Neelakantan, Pranav Shyam, Girish Sastry, Amanda Askell, et al. Language models are few-shot learners. arXiv preprint arXiv:2005.14165, 2020.

Generally, an attention mechanism maps a query and a set of key-value pairs to an output, where the query, keys, and values are all vectors. The output is computed as a weighted sum of the values, where the weight assigned to each value is computed by a compatibility function, e.g. a dot product or scaled dot product, of the query with the corresponding key.

Generally, a self-attention mechanism is configured to relate different positions in the same sequence to determine a transformed version of the sequence as an output. For example the attention layer input may comprise a vector for each element of the input sequence. These vectors provide an input to the self-attention mechanism and are used by the self-attention mechanism to determine a new representation of the same sequence for the attention layer output, which similarly comprises a vector for each element of the input sequence. An output of the self-attention mechanism may be used as the attention layer output, or it may be processed by one or more feed-forward layers, skip connections, or normalization operations to provide the attention layer output.

In some implementations the attention mechanism is configured to apply each of a query transformation e.g. defined by a matrix *W^{Q}*, a key transformation e.g. defined by a matrix *W^{K}*, and a value transformation e.g. defined by a matrix *W^{V},* to the attention layer input which is the input data *X* to the attention layer, to derive a query matrix *Q* = *XW^{Q}* that includes a respective query for each vector in the input sequence. key matrix *K* = *XW^{K}* that includes a respective key for each vector in the input sequence, and value matrix *V = XW^{V}* that includes a respective value for each vector in the input sequence, which are used to determine an attended sequence for the output. For example the attention mechanism may be a dot product attention mechanism applied by applying each query vector to each key vector to determine respective weights for each value vector, then combining the value vectors using the respective weights to determine the self-attention layer output for each element of the input sequence. The self-attention layer output may be scaled by a scaling factor e.g. by the square root of the dimensions of the queries and keys, to implement scaled dot product attention. Thus, for example, an output of the attention mechanism may be determined as $softmax \left(\frac{{QK}^{T}}{\sqrt{d}}\right) V$ where d is a dimension of the key (and value) vector. In another implementation the attention mechanism may comprise an "additive attention" mechanism that computes the compatibility function using a feed-forward network with a hidden layer. The output of the attention mechanism may be further processed by one or more fully-connected, feed forward neural network layers.

The attention mechanism may implement multi-head attention, that is, it may apply multiple different attention mechanisms in parallel. The outputs of these may then be combined, e.g. concatenated, with a learned linear transformation applied to reduce to the original dimensionality if necessary.

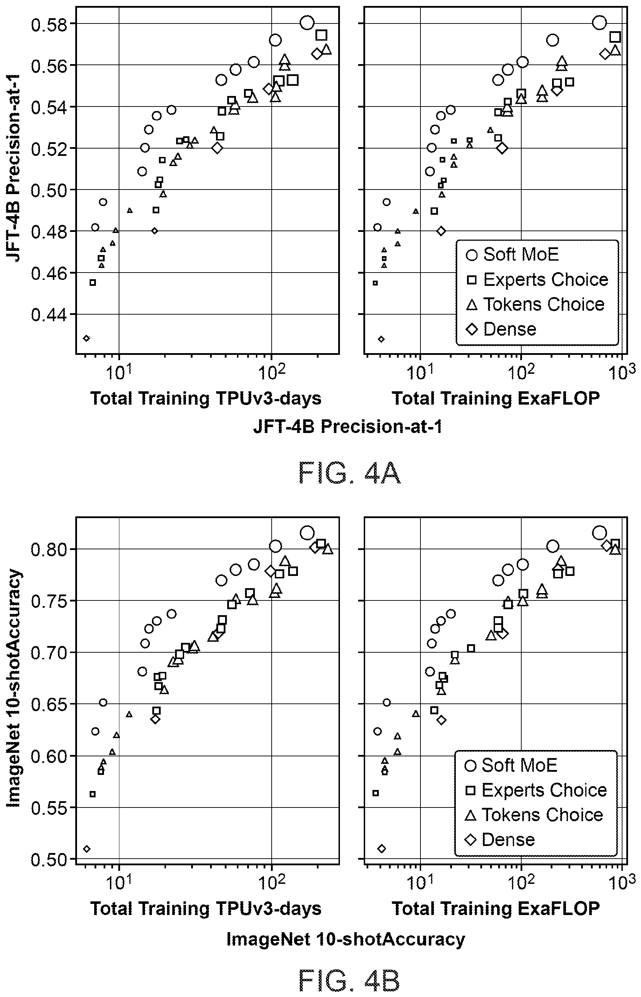

FIG. 4A illustrates a comparison of performance of a soft MoE model and other models including a Vision Transformer ("ViT" or "dense") model, a token-choice routing-based model (or "Tokens Choice" model), and an expert-choice routing-based model (or "Experts Choice" model) on an image classification task using the upstream validation precision-at-1 metric. FIG. 4B illustrates a comparison of performance of the soft MoE model and the ViT model, the token-choice routing-based model, and the expert-choice routing-based model using the ImageNet 10-shot accuracy metric. All of the model are pre-trained on JFT-4B which is a proprietary dataset that contains more than 4 billion images, covering 29,000 classes. The performance of each model is represented by a marker (e.g., a Pareto frontier point) on a Pareto frontier curve. Larger marker sizes indicate larger models. The training cost is represented by TPU-v3 training time.

As shown in FIGS. 4A and 4B, in both cases when the performance of these models are evaluated in the upstream validation precision-at-1 metric on JFT-4B and the ImageNet 10-shot accuracy metric, the soft MoE model dominates both of the ViT model and popular MoE models (Experts Choice and Tokens Choice models) on the training cost and performance Pareto frontier. These results show that soft MoE routing strongly outperforms dense and other sparse approaches for any given FLOPs or time budget.

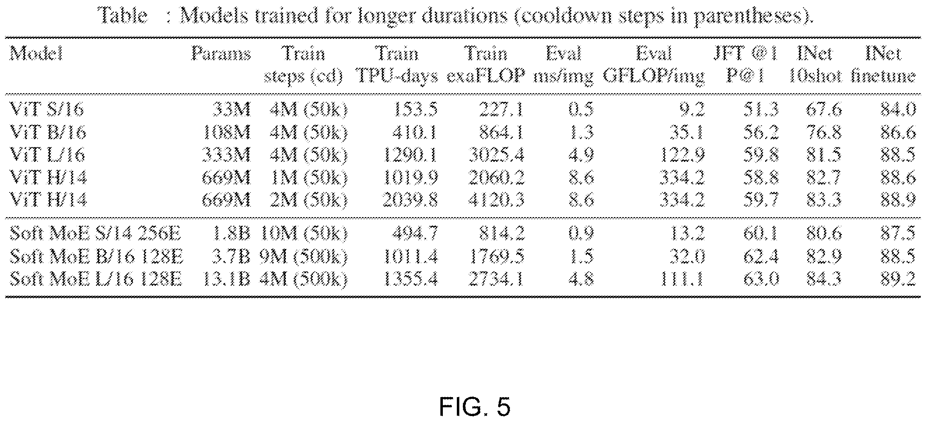

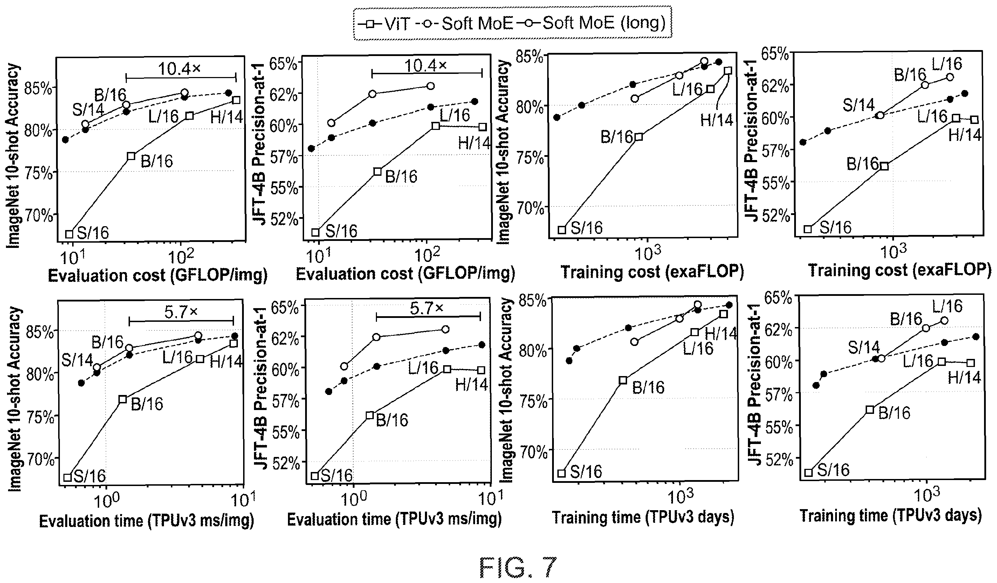

FIG. 5 is a table that shows different Vision Transformer (ViT) models and soft MoE models. For each model, the table lists the number of parameters, training steps, training TPU-days, computation units (FLOPs) and performance evaluations of the model based on different metrics (e.g., ms/img, GFLOP/img, JFT, ImageNet 10-shot, and ImageNet-finetuning). These performance evaluations will be described in further detail below with reference to FIG. 7.

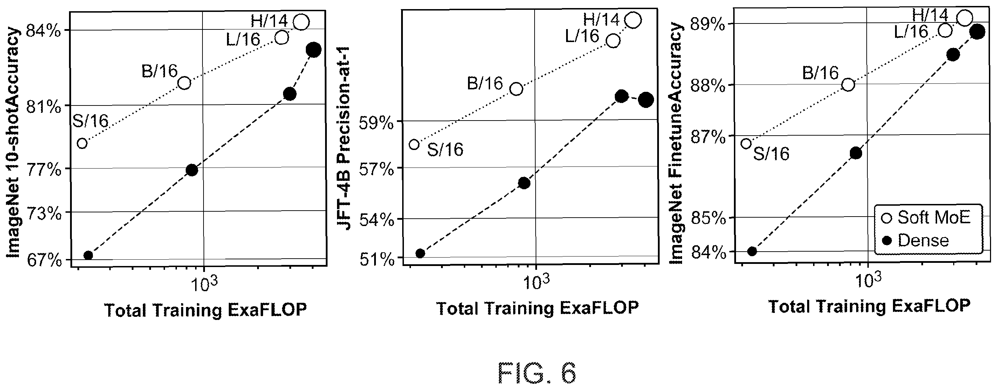

FIG. 6 shows performance of some ViT (Dense) models (i.e., ViT S/16, B/16, L/16, H/16) and soft MoE models (i.e., Soft MoE S/14, B/16, and L/16) listed in the table in FIG. 5 with long training durations. The number of parameters of each model is in the range of 1 billion to 54 billion parameters. All models were trained for 4 million steps, except for H/14, which was trained for 2 million steps for cost reasons.

More specifically, FIG. 6 shows the JFT-4B precision, ImageNet 10-shot accuracy, and the ImageNet finetuning accuracy for Soft MoE and ViT versus training cost. Soft MoE substantially outperforms dense ViT models for a given compute budget. For example, the Soft MoE S/16 performs better than ViT B/16 on JFT and 10-shot ImageNet, and it also improves finetuning scores on the full ImageNet data, even though its training (and inference) cost is significantly smaller. Similarly, Soft MoE B/16 outperforms ViT L/16 upstream, and only lags
0.5 behind after finetuning while being 3 times faster and requiring almost 4 times fewer FLOPs. Finally, the Soft MoE L/16 model outperforms the dense H/14 one while again being around 3 times faster in terms of training and inference step time.

FIG. 7 shows performance of ViT (Dense) models (i.e., ViT S/16, B/16, L/16, H/16) and soft MoE models (i.e., Soft MoE S/14, B/16, and L/16) when they are optimized for inference speed.

These models have been trained up to 9 million steps to become models of high quality with low inference cost. Even after additional (over) training, the overall training time with respect to larger ViT models is similar or smaller. For these runs, longer cooldowns (linear learning rate decay) works well for Soft MoE. Therefore, the cooldown steps for Soft MoE can be increased from 50k steps to 500k steps.

As shown in Figure 7 and the table of FIG. 5, the Soft MoE B/16 model which is trained for 1k TPUv3 days matches or outperforms the ViT H/14 which is trained on a similar budget, and is 10 times cheaper at inference in FLOPs (32 vs. 334 GFLOPS/img) and more than 5 times cheaper in wall-clock time (1.5 vs. 8.6 ms/img). The Soft MoE B/16 model matches the ViT H/14 model's performance when ViT-H/14's training budget is doubled (to 2k TPU-days). Soft MoE L/16 outperforms all ViT models while being almost 2 times faster at inference than ViT H/14 (4.8 vs. 8.6 ms/img).

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non volatile memory, media and memory devices, including by way of example semiconductor memory devices, e.g., EPROM, EEPROM, and flash memory devices: magnetic disks, e.g., internal hard disks or removable disks; magneto optical disks; and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and compute-intensive parts of machine learning training or production, i.e., inference, workloads.

Machine learning models can be implemented and deployed using a machine learning framework, .e.g., a TensorFlow framework, a Microsoft Cognitive Toolkit framework, an Apache Singa framework, or an Apache MXNet framework.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention, which is defined by the appended claims, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. As one example, the processes depicted in the accompanying figures do not necessarily require the particular order shown, or sequential order, to achieve desirable results. In some cases, multitasking and parallel processing may be advantageous.

## Claims

1. A computing system comprising a neural network (150) that is configured to process a network input (102) and to generate a network output (120) for the network input (102), the neural network (150) comprising a sequence of one or more network blocks, the sequence comprising one or more expert network blocks, at least one (110-2) of the one or more expert network blocks being configured to perform operations comprising:
obtaining a block input (103) that represents an intermediate representation of the network input (102), the block input (103) comprising a plurality of input tokens;
generating a plurality of input slots from the plurality of input tokens, wherein each input slot is associated with a respective set of dispatch weights and is generated by combining all of the plurality of input tokens in accordance with the respective set of dispatch weights;
for each of a plurality of expert subnetworks (106) of the at least one expert network block (110-2), performing operations comprising:
processing a respective subset of the plurality of input slots using the expert subnetwork to generate, for each input slot in the respective subset, a respective output slot for the input slot; wherein the number of input slots processed by the expert subnetwork is less than the total number of input tokens;
updating the plurality of input tokens using the output slots to generate a plurality of output tokens;
generating a block output (112) from at least the plurality of output tokens; and
wherein the operations performed for at least some of the plurality of expert subnetworks (106) are executed in parallel, by a computing device configured for parallel processing, and/or by respective different devices of the system.

2. The system of claim 1, wherein updating the plurality of input tokens using the output slots comprises:
for each of the plurality of input tokens, generating a respective output token, wherein each input token is associated with a respective set of combine weights, and wherein generating the respective output token for each input token comprises:
combining the output slots generated by the plurality of expert subnetworks (106) in accordance with the respective set of combine weights associated with the input token.

3. The system of claim 1 or 2, wherein each expert subnetwork is configured to process a same number of input slots.

4. The system of any one of claims 1-3, wherein generating the plurality of input slots from the plurality of input tokens comprises computing: ${X}^{˜} = {D}^{T} X ,$ where *X̃* represents the plurality of input slots, *D^{T}* is a transpose of a dispatch weight matrix *D* that includes a respective set of dispatch weights for each input token, and $X ∈ {ℝ}^{m \times d}$ represents the plurality of input tokens, where *m* is the number of input tokens and *d* is their dimension.

5. The system of claim 4, wherein the dispatch weight matrix *D* is generated by applying a softmax over columns of a routing weight matrix *L* as follows: ${D}_{i , j} = \frac{exp \left({L}_{i , j}\right)}{{\sum }_{i ′ = 1}^{m} exp \left({L}_{i ′ , j}\right)} ,$ wherein *i* and *i'* are row indices of the dispatch weights matrix D and the routing weight matrix *L, j* is the column index of the dispatch weight matrix D and the routing weight matrix *L.*

6. The system of claim 5, wherein the routing weight matrix *L* is a product of the plurality of input tokens *X* and a set of trainable parameters *ϕ*.

7. The system of any one of claims 1-6, wherein for each of the plurality of expert subnetworks (106) of the at least one expert network block (110-2), processing the respective subset of the plurality of input slots using the expert subnetwork to generate, for each input slot in the respective subset, the respective output slot for the input slot comprises computing: ${{Y}^{˜}}_{i} = {f}_{\left⌊i/p\right⌋} \left({{X}^{˜}}_{i}\right) ,$ where *f* is the expert subnetwork, *X̃ᵢ* represents each input slot in the respective subset of *p* input slots processed by the expert subnetwork, and *Ỹᵢ* is the respective output slot generated by the expert subnetwork for the input slot *X̃ᵢ.*

8. The system of any one of claims 1-7, wherein updating the plurality of input tokens using the output slots to generate the plurality of output tokens comprises computing: $Y = C {Y}^{˜} ,$ where *Ỹ* represents the output slots, *C* is a combine weight matrix that includes a respective set of combine weights for each input token, and *Y* represents the plurality of output tokens.

9. The system of claim 8, wherein the combine weight matrix *C* is generated by applying a softmax over rows of a routing weight matrix *L* as follows: ${C}_{i , j} = \frac{exp \left({L}_{i , j}\right)}{{\sum }_{j ′ = 1}^{n . p} exp \left({L}_{i , j ′}\right)} ,$ wherein *i* is the row index of the combine weight matrix *C* and the routing weight matrix *L,j* and *j'* are column indices of the combine weight matrix *C* and the routing weight matrix *L.*

10. The system of claim 9, wherein the routing weight matrix *L* is a product of the plurality of input tokens X and a set of trainable parameters *ϕ*.

11. The system of any one of claims 1-10, wherein generating the block output from at least the plurality of output tokens comprises:
applying a residual connection to the plurality of output tokens to generate the block output (112).

12. A computer-implemented method performed by a computing system comprising at least one of one or more expert network blocks, the one or more expert network blocks comprised in a sequence of one or more network blocks of a neural network, the neural network configured to process a network input and to generate a network output for the network input, the method comprising:
obtaining (302) a block input that represents an intermediate representation of the network input, the block input comprising a plurality of input tokens;
generating (304) a plurality of input slots from the plurality of input tokens, wherein each input slot is associated with a respective set of dispatch weights and is generated by combining all of the plurality of input tokens in accordance with the respective set of dispatch weights;
for each of a plurality of expert subnetworks of the at least one expert network block, performing operations comprising:
processing (306) a respective subset of the plurality of input slots using the expert subnetwork to generate, for each input slot in the respective subset, a respective output slot for the input slot; wherein the number of input slots processed by the expert subnetwork is less than the total number of input tokens;
updating (308) the plurality of input tokens using the output slots to generate a plurality of output tokens;
generating (310) a block output from at least the plurality of output tokens; and
wherein the operations performed for at least some of the plurality of expert subnetworks are executed in parallel, by a computing device configured for parallel processing, and/or by respective different devices of the system.

13. The method of claim 12, wherein the method further comprises the operations carried out by the system of any one of claims 2-11.

14. One or more computer storage media storing instructions that when executed by a computing system according to any one of claims 1-11 causes the computing system to perform the operations carried out by the system of any one of claims 1-11.

## Patentansprüche

1. Rechensystem, umfassend ein neuronales Netzwerk (150), das dazu konfiguriert ist, eine Netzwerkeingabe (102) zu verarbeiten und für die Netzwerkeingabe (102) eine Netzwerkausgabe (120) zu erzeugen, wobei das neuronale Netzwerk (150) eine Sequenz von einem oder mehreren Netzwerkblöcken umfasst, wobei die Sequenz einen oder mehrere Expertennetzwerkblöcke umfasst, wobei mindestens einer (110-2) des einen oder der mehreren Expertennetzwerkblöcke dazu konfiguriert ist, Operationen auszuführen, die Folgendes umfassen:
Erhalten einer Blockeingabe (103), die eine Zwischendarstellung der Netzwerkeingabe (102) darstellt, wobei die Blockeingabe (103) eine Mehrzahl von Eingabetoken umfasst;
Erzeugen einer Mehrzahl von Eingabeslots aus der Mehrzahl von Eingabetoken, wobei jeder Eingabeslot einem jeweiligen Satz von Dispatch-Gewichten zugeordnet ist und durch Kombinieren aller Eingabetoken der Mehrzahl gemäß dem jeweiligen Satz von Dispatch-Gewichten erzeugt wird;
für jedes einer Mehrzahl von Expertenteilnetzwerken (106) des mindestens einen Expertennetzwerkblocks (110-2), Ausführen von Operationen, die Folgendes umfassen:
Verarbeiten einer jeweiligen Teilmenge der Mehrzahl von Eingabeslots unter Verwendung des Expertenteilnetzwerks, um für jeden Eingabeslot in der jeweiligen Teilmenge einen jeweiligen Ausgabeslot für den Eingabeslot zu erzeugen; wobei die Anzahl der von dem Expertenteilnetzwerk verarbeiteten Eingabeslots kleiner ist als die Gesamtzahl der Eingabetoken;
Aktualisieren der Mehrzahl von Eingabetoken unter Verwendung der Ausgabeslots, um eine Mehrzahl von Ausgabetoken zu erzeugen;
Erzeugen einer Blockausgabe (112) aus mindestens der Mehrzahl von Ausgabetoken; und
wobei die für mindestens einige der Mehrzahl von Expertenteilnetzwerken (106) ausgeführten Operationen parallel ausgeführt werden, durch eine für Parallelverarbeitung konfigurierte Rechenvorrichtung und/oder durch jeweilige unterschiedliche Vorrichtungen des Systems.

2. System nach Anspruch 1, wobei das Aktualisieren der Mehrzahl von Eingabetoken unter Verwendung der Ausgabeslots Folgendes umfasst:
für jedes der Mehrzahl von Eingabetoken, Erzeugen eines jeweiligen Ausgabetokens, wobei jedes Eingabetoken einem jeweiligen Satz von Kombinationsgewichten zugeordnet ist und wobei das Erzeugen des jeweiligen Ausgabetokens für jedes Eingabetoken Folgendes umfasst:
Kombinieren der von der Mehrzahl von Expertenteilnetzwerken (106) erzeugten Ausgabeslots gemäß dem jeweiligen Satz von Kombinationsgewichten, der dem Eingabetoken zugeordnet ist.

3. System nach Anspruch 1 oder 2, wobei jedes Expertenteilnetzwerk dazu konfiguriert ist, eine gleiche Anzahl von Eingabeslots zu verarbeiten.

4. System nach einem der Ansprüche 1-3, wobei das Erzeugen der Mehrzahl von Eingabeslots aus der Mehrzahl von Eingabetoken das Berechnen von Folgendem umfasst: ${X}^{˜} = {D}^{T} X ,$ wobei *X̃* die Mehrzahl von Eingabeslots darstellt, *D^{T}* eine Transponierte einer Dispatch-Gewichtsmatrix *D* ist, die einen jeweiligen Satz von Dispatch-Gewichten für jedes Eingabetoken enthält, und $X ∈ {ℝ}^{m \times d}$ die Mehrzahl von Eingabetoken darstellt, wobei *m* die Anzahl der Eingabetoken ist und *d* deren Dimension ist.

5. System nach Anspruch 4, wobei die Dispatch-Gewichtsmatrix *D* durch Anwenden einer Softmax-Funktion über Spalten einer Routing-Gewichtsmatrix *L* wie folgt erzeugt wird: ${D}_{i , j} = \frac{exp \left({L}_{i , j}\right)}{{\sum }_{i ′ = 1}^{m} exp \left({L}_{i ′ , j}\right)} ,$ wobei *i* und *i'* Zeilenindizes der Dispatch-Gewichtsmatrix D und der Routing-Gewichtsmatrix *L* sind, *j* der Spaltenindex der Dispatch-Gewichtsmatrix D und der Routing-Gewichtsmatrix *L* ist.

6. System nach Anspruch 5, wobei die Routing-Gewichtsmatrix *L* ein Produkt der Mehrzahl von Eingabetoken *X* und einer Menge trainierbarer Parameter *ϕ* ist.

7. System nach einem der Ansprüche 1-6, wobei für jedes der Mehrzahl von Expertenteilnetzwerken (106) des mindestens einen Expertennetzwerkblocks (110-2) das Verarbeiten der jeweiligen Teilmenge der Mehrzahl von Eingabeslots unter Verwendung des Expertenteilnetzwerks, um für jeden Eingabeslot in der jeweiligen Teilmenge den jeweiligen Ausgabeslot für den Eingabeslot zu erzeugen, das Berechnen von Folgendem umfasst: ${{Y}^{˜}}_{i} = {f}_{\left⌊i/p\right⌋} \left({{X}^{˜}}_{i}\right)$ wobei *f* das Expertenteilnetzwerk ist, *X̃ᵢ* jeden Eingabeslot in der jeweiligen Teilmenge von *p* Eingabeslots darstellt, die vom Expertenteilnetzwerk verarbeitet werden, und *Ỹᵢ* der jeweilige Ausgabeslot ist, der vom Expertenteilnetzwerk für den Eingabeslot *X̃ᵢ* erzeugt wird.

8. System nach einem der Ansprüche 1-7, wobei das Aktualisieren der Mehrzahl von Eingabetoken unter Verwendung der Ausgabeslots, um die Mehrzahl von Ausgabetoken zu erzeugen, das Berechnen von Folgendem umfasst: $Y = C {Y}^{˜} ,$ wobei *Ỹ* die Ausgabeslots darstellt, *C* eine Kombinationsgewichtsmatrix ist, die einen jeweiligen Satz von Kombinationsgewichten für jedes Eingabetoken enthält, und *Y* die Mehrzahl von Ausgabetoken darstellt.

9. System nach Anspruch 8, wobei die Kombinationsgewichtsmatrix *C* durch Anwenden einer Softmax-Funktion über Zeilen einer Routing-Gewichtsmatrix *L* wie folgt erzeugt wird: ${C}_{i , j} = \frac{exp \left({L}_{i , j}\right)}{{\sum }_{j ′ = 1}^{n . p} exp \left({L}_{i , j ′}\right)} ,$ wobei *i* der Zeilenindex der Kombinationsgewichtsmatrix *C* und der Routing-Gewichtsmatrix *L* ist, *j* und *j*' die Spaltenindizes der Kombinationsgewichtsmatrix *C* und der Routing-Gewichtsmatrix *L* sind.

10. System nach Anspruch 9, wobei die Routing-Gewichtsmatrix *L* ein Produkt der Mehrzahl von Eingabetoken *X* und einer Menge trainierbarer Parameter *ϕ* ist.

11. System nach einem der Ansprüche 1-10, wobei das Erzeugen der Blockausgabe aus mindestens der Mehrzahl von Ausgabetoken Folgendes umfasst:
Anwenden einer Residualverbindung auf die Mehrzahl von Ausgabetoken, um die Blockausgabe (112) zu erzeugen.

12. Computerimplementiertes Verfahren, das von einem Rechensystem durchgeführt wird, das mindestens einen von einem oder mehreren Expertennetzwerkblöcken umfasst, wobei der eine oder die mehreren Expertennetzwerkblöcke in einer Sequenz von einem oder mehreren Netzwerkblöcken eines neuronalen Netzwerks enthalten sind, wobei das neuronale Netzwerk dazu konfiguriert ist, eine Netzwerkeingabe zu verarbeiten und eine Netzwerkausgabe für die Netzwerkeingabe zu erzeugen, wobei das Verfahren Folgendes umfasst:
Erhalten (302) einer Blockeingabe, die eine Zwischendarstellung der Netzwerkeingabe darstellt, wobei die Blockeingabe eine Mehrzahl von Eingabetoken umfasst;
Erzeugen (304) einer Mehrzahl von Eingabeslots aus der Mehrzahl von Eingabetoken, wobei jeder Eingabeslot einem jeweiligen Satz von Dispatch-Gewichten zugeordnet ist und durch Kombinieren aller Eingabetoken der Mehrzahl gemäß dem jeweiligen Satz von Dispatch-Gewichten erzeugt wird;
für jedes einer Mehrzahl von Expertenteilnetzwerken des mindestens einen Expertennetzwerkblocks, Ausführen von Operationen, die Folgendes umfassen:
Verarbeiten (306) einer jeweiligen Teilmenge der Mehrzahl von Eingabeslots unter Verwendung des Expertenteilnetzwerks, um für jeden Eingabeslot in der jeweiligen Teilmenge einen jeweiligen Ausgabeslot für den Eingabeslot zu erzeugen; wobei die Anzahl der von dem Expertenteilnetzwerk verarbeiteten Eingabeslots kleiner ist als die Gesamtzahl der Eingabetoken;
Aktualisieren (308) der Mehrzahl von Eingabetoken unter Verwendung der Ausgabeslots, um eine Mehrzahl von Ausgabetoken zu erzeugen;
Erzeugen (310) einer Blockausgabe aus mindestens der Mehrzahl von Ausgabetoken; und
wobei die für mindestens einige der Mehrzahl von Expertenteilnetzwerken ausgeführten Operationen parallel ausgeführt werden, durch eine für Parallelverarbeitung konfigurierte Rechenvorrichtung und/oder durch jeweilige unterschiedliche Vorrichtungen des Systems.

13. Verfahren nach Anspruch 12, wobei das Verfahren ferner die von dem System nach einem der Ansprüche 2-11 durchgeführten Operationen umfasst.

14. Ein oder mehrere Computerspeichermedien, die Anweisungen speichern, die bei Ausführung durch ein Rechensystem nach einem der Ansprüche 1-11 das Rechensystem veranlassen, die von dem System nach einem der Ansprüche 1-11 durchgeführten Operationen auszuführen.

## Revendications

1. Système informatique comprenant un réseau neuronal (150) configuré pour traiter une entrée réseau (102) et générer une sortie réseau (120) pour l'entrée réseau (102), le réseau neuronal (150) comprenant une séquence d'un ou plusieurs blocs réseau, la séquence comprenant un ou plusieurs blocs réseau d'experts, au moins un (110-2) des un ou plusieurs blocs réseau d'experts étant configuré pour réaliser des opérations comprenant :
l'obtention d'une entrée de bloc (103) qui représente une représentation intermédiaire de l'entrée réseau (102), l'entrée de bloc (103) comprenant une pluralité de jetons d'entrée ;
la génération d'une pluralité d'emplacements d'entrée à partir de la pluralité de jetons d'entrée, dans lequel chaque emplacement d'entrée est associé à un ensemble respectif de poids de répartition et est généré en combinant l'ensemble de la pluralité de jetons d'entrée conformément à l'ensemble respectif de poids de répartition ;
pour chacun d'une pluralité de sous-réseaux d'experts (106) de l'au moins un bloc de réseau d'experts (110-2), la réalisation des opérations comprenant :
le traitement d'un sous-ensemble respectif de la pluralité d'emplacements d'entrée à l'aide du sous-réseau d'experts afin de générer, pour chaque emplacement d'entrée du sous-ensemble respectif, un emplacement de sortie respectif pour l'emplacement d'entrée ; dans lequel le nombre d'emplacements d'entrée traités par le sous-réseau d'experts est inférieur au nombre total de jetons d'entrée ;
la mise à jour de la pluralité de jetons d'entrée à l'aide des emplacements de sortie pour générer une pluralité de jetons de sortie ;
la génération d'une sortie de bloc (112) à partir d'au moins la pluralité de jetons de sortie ; et
dans lequel les opérations réalisées pour au moins une partie de la pluralité de sous-réseaux d'experts (106) sont exécutées en parallèle, par un dispositif informatique configuré pour le traitement parallèle, et/ou par les différents dispositifs respectifs du système.

2. Système selon la revendication 1, dans lequel la mise à jour de la pluralité de jetons d'entrée à l'aide des emplacements de sortie comprend :
pour chacun de la pluralité de jetons d'entrée, la génération d'un jeton de sortie respectif, dans lequel chaque jeton d'entrée est associé à un ensemble respectif de poids combinés, et dans lequel la génération du jeton de sortie respectif pour chaque jeton d'entrée comprend :
la combinaison des emplacements de sortie générés par la pluralité de sous-réseaux d'experts (106) conformément à l'ensemble respectif de poids combinés associés au jeton d'entrée.

3. Système selon la revendication 1 ou 2, dans lequel chaque sous-réseau d'experts est configuré pour traiter un même nombre d'emplacements d'entrée.

4. Système selon l'une quelconque des revendications 1 à 3, dans lequel la génération de la pluralité d'emplacements d'entrée à partir de la pluralité de jetons d'entrée comprend le calcul : ${X}^{˜} = {D}^{T} X ,$ où *X̃* représente la pluralité d'emplacements d'entrée, *D^{T}* est une transposée d'une matrice de poids de répartition *D* qui comporte un ensemble respectif de poids de répartition pour chaque jeton d'entrée, et $X ∈ {ℝ}^{m \times d}$ représente la pluralité de jetons d'entrée, où *m* est le nombre de jetons d'entrée et *d* est leur dimension.

5. Système selon la revendication 4, dans lequel la matrice de poids de répartition *D* est générée en appliquant une fonction softmax aux colonnes d'une matrice de poids de routage *L* comme suit : ${D}_{i , j} = \frac{exp \left({L}_{i , j}\right)}{{\sum }_{i ′ = 1}^{m} exp \left({L}_{i ′ , j}\right)} ,$ dans lequel *i* et *i'* sont les indices de ligne de la matrice de poids de répartition D et de la matrice de poids de routage *L, j* est l'indice de colonne de la matrice de poids de répartition D et de la matrice de poids de routage *L.*

6. Système selon la revendication 5, dans lequel la matrice de poids de routage *L* est un produit de la pluralité de jetons d'entrée *X* et d'un ensemble de paramètres entraînables *ϕ*.

7. Système selon l'une quelconque des revendications 1 à 6, dans lequel pour chacun de la pluralité de sous-réseaux d'experts (106) de l'au moins un bloc de réseau d'experts (110-2), le traitement du sous-ensemble respectif de la pluralité d'emplacements d'entrée à l'aide du sous-réseau d'experts pour générer, pour chaque emplacement d'entrée du sous-ensemble respectif, l'emplacement de sortie respectif pour l'emplacement d'entrée comprend le calcul : ${{Y}^{˜}}_{i} = {f}_{\left⌊i/p\right⌋} \left({{X}^{˜}}_{i}\right)$ où *f* est le sous-réseau d'experts, *X̃ᵢ* représente chaque emplacement d'entrée dans le sous-ensemble respectif de *p* emplacements d'entrée traités par le sous-réseau d'experts, et *Ỹᵢ* est l'emplacement de sortie respectif généré par le sous-réseau d'experts pour l'emplacement d'entrée *X̃ᵢ.*

8. Système selon l'une quelconque des revendications 1 à 7, dans lequel la mise à jour de la pluralité de jetons d'entrée à l'aide des emplacements de sortie pour générer la pluralité de jetons de sortie comprend le calcul : $Y = C {Y}^{˜} ,$ où *Ỹ* représente les emplacements de sortie, *C* est une matrice de poids combinés qui comporte un ensemble respectif de poids combinés pour chaque jeton d'entrée, et *Y* représente la pluralité de jetons de sortie.

9. Système selon la revendication 8, dans lequel la matrice de poids combinés *C* est générée en appliquant une fonction softmax aux lignes d'une matrice de poids de routage *L* comme suit : ${C}_{i , j} = \frac{exp \left({L}_{i , j}\right)}{{\sum }_{j ′ = 1}^{n . p} exp \left({L}_{i , j ′}\right)} ,$ dans lequel *i* est l'indice de ligne de la matrice de poids combinés *C* et de la matrice de poids de routage *L, j* et *j'* sont les indices de colonne de la matrice de poids combinés C et de la matrice de poids de routage *L.*

10. Système selon la revendication 9, dans lequel la matrice de poids de routage *L* est un produit de la pluralité de jetons d'entrée *X* et d'un ensemble de paramètres entraînables *ϕ*.

11. Système selon l'une quelconque des revendications 1 à 10, dans lequel la génération de la sortie de bloc à partir d'au moins la pluralité de jetons de sortie comprend :
l'application d'une connexion résiduelle à la pluralité de jetons de sortie pour générer la sortie de bloc (112).

12. Procédé mis en œuvre par ordinateur réalisé par un système informatique comprenant au moins l'un d'un ou plusieurs blocs de réseau d'experts, les un ou plusieurs blocs de réseau d'experts étant inclus dans une séquence d'un ou plusieurs blocs de réseau d'un réseau neuronal, le réseau neuronal étant configuré pour traiter une entrée réseau et générer une sortie réseau pour l'entrée réseau, le procédé comprenant :
l'obtention (302) d'une entrée de bloc qui représente une représentation intermédiaire de l'entrée réseau, l'entrée de bloc comprenant une pluralité de jetons d'entrée ;
la génération (304) d'une pluralité d'emplacements d'entrée à partir de la pluralité de jetons d'entrée, dans lequel chaque emplacement d'entrée est associé à un ensemble respectif de poids de répartition et est généré en combinant l'ensemble de la pluralité de jetons d'entrée conformément à l'ensemble respectif de poids de répartition ;
pour chacun d'une pluralité de sous-réseaux d'experts de l'au moins un bloc de réseau d'experts, la réalisation des opérations comprenant :
le traitement (306) d'un sous-ensemble respectif de la pluralité d'emplacements d'entrée à l'aide du sous-réseau d'experts afin de générer, pour chaque emplacement d'entrée du sous-ensemble respectif, un emplacement de sortie respectif pour l'emplacement d'entrée ; dans lequel le nombre d'emplacements d'entrée traités par le sous-réseau d'experts est inférieur au nombre total de jetons d'entrée ;
la mise à jour (308) de la pluralité de jetons d'entrée à l'aide des emplacements de sortie pour générer une pluralité de jetons de sortie ;
la génération (310) d'une sortie de bloc à partir d'au moins la pluralité de jetons de sortie ; et
dans lequel les opérations réalisées pour au moins une partie de la pluralité de sous-réseaux d'experts sont exécutées en parallèle, par un dispositif informatique configuré pour le traitement parallèle, et/ou par les différents dispositifs respectifs du système.

13. Procédé selon la revendication 12, dans lequel le procédé comprend en outre les opérations effectuées par le système selon l'une quelconque des revendications 2 à 11.

14. Un ou plusieurs supports de stockage informatique stockant des instructions qui lorsqu'elles sont exécutées par un système selon l'une quelconque des revendications 1 à 11 amènent le système informatique à réaliser les opérations effectuées par le système selon l'une quelconque des revendications 1 à 11.
